# EUROPEAN PATENT SPECIFICATION

(11) **EP 2 936 997 B1**
(45) Date of publication and mention of the grant of the patent: **16.10.2019**
(21) Application number: 15164414.3
(22) Date of filing: 21.04.2015
(51) Int. Cl.: A23L 33/10, A23L 29/00, C12C 3/00, C12C 3/08, C12C 3/12

(54) **ISOMERISATION OF SPENT HOP MATERIAL**
ISOMERISIERUNG VON VERBRAUCHTEM HOPFENMATERIAL
ISOMÉRISATION DE MATÉRIAU DE DÉCHET DE HOUBLON

(30) Priority: 22.04.2014 GB 201407084
(43) Date of publication of application: 28.10.2015
(73) Proprietor: Yakima Chief, Inc., Sunnyside, WA 98944 (US)
(72) Inventor: Janssens, Philippe, 1300 Wavre (BE)
(74) Representative: IPLodge bvba

(56) References cited:
- EP-A1- 0 363 023
- WO-A1-03/090555
- WO-A1-2011/135032
- WO-A1-2012/172147
- WO-A2-2009/023710

## Description

### Field of the invention

This invention relates to methods of isomerisation of X (xanthohumol) into IX (isoxanthohumol) in spent hop products. The invention further relates to spent hop material comprising elevated levels of IX (isoxanthohumol).

### Background of the invention

The cultivated hop (*Humulus Lupulus* L.) is a dioecious plant of the *Cannabinaceae* family (order *Urticales*)*,* mainly used for the brewing industry. It is well known that hops have additional advantages and are used for their pharmaceutical properties, such as sedative, anti-inflammatory, antimicrobial, anti-cancer and estrogenic.

Hop contains an important diversity of organic compounds, however the major components present in hop are given below in % w/w:

| | | | |
|---|---|---|---|
| Hop resins: | 4 - 27 | Lipids and fatty acids | 1 - 5 |
| Hop oil: | 0.5 - 3.0 | Pectins | 2 |
| Hop polyphenols and tannins: | 3 - 6 | Ash-salts | 10 |
| Monosaccharides: | 2 | Cellulose - lignins | 40 - 50 |
| Amino acids | 0.1 | Water | 8 - 12 |
| Proteins | 15 | | |

There are three important classes of compounds that present some industrial interests:
- The hop resins, which are used in the brewing industry to communicate the bitterness of the beer and which are also recognized for their bacteriostatic effect.
- The hop oils, which are used to contribute to the flavour of the beer, and which are also used in the cosmetic industry.
- The hop polyphenols, which represent a vast class of compounds with widely varying structural characteristics, but having in common the presence of one or more phenolic functional groups. Their composition depends greatly on the hop variety, the cultivation area, the harvesting technique and the degree of ageing.

They comprise phenolic acids, flavanoids, flavonols, flavonol glycosides, phytoalexines and prenylflavonoids. These prenylflavonoids are generally divided in two sub-classes, the prenylchalcones, containing xanthohumol (X) and desmethylxanthohumol (DMX), which are precursors of the prenylflavanones, containing isoxanthohumol (IX), 8-prenylnaringenin (8-PN), 6-prenylnaringenin (6-PN), 6,8-diprenylnaringenin and 8-geranylnaringenin.

The estrogenic activity of hop is well known and is mainly due to the presence of 8-PN, whereas the other prenylflavanones play a minor contribution and the prenylchalcones have no action.

EP0311330 discloses a process for the stabilisation and isomerisation of alpha-acids in hops into iso-alpha-acids by mixing comminuted hops with calcium or magnesium oxide or hydroxide and forming pellets of this mixture. The pellets are packed in the absence of oxygen, sealed and heated between 40 to 55 °C for at least one day.

WO02085393 discloses a hop extract obtained from the female hop cones of certain varieties of hops, comprising the following prenylflavanoids: xanthohumol, isoxanthohumol and 8-prenylnaringenin, in defined weight proportions. It is also known that at temperature between 40 °C and 60 °C, the isomerisation of the precursors of the prenylflavanones, namely the prenylchalcones is accelerated. Following the isomerisation, X is converted into IX and DMX is converted into a mixture of 8-PN and 6-PN.

WO2005058336 discloses a method for the production of a hop extract which is enriched in 8-prenylnaringenin with respect to 6-prenylnaringenin. The method comprises the steps of subjecting hops or a hop product to: (1) an isomerisation in the presence of water as a solvent and in the presence of an amount of a base (>0.1 %w/v KOH) and (2) to at least one extraction (liquid or supercritical CO₂). The hop extract comprises a mixture of X and 8-PN, in a defined ratio.

The composition of hop is relatively low in 8-PN, and several methods have been developed with limited success to increase the content in 8-PN of the extract. WO2009023710 discloses a method for separating xanthohumol and isoxanthohumol in a solution of a hop extract by differential precipitation, in order to remove isoxanthohumol and chlorophyll. The enriched xanthohumol fraction was dried and incubated at about 75 °C during 120 hours. The conversion of desired xanthohumol to unwanted isoxanthumol was limited (isoxanthohumol /xanthohumol (IX/XN) of 0.03).

WO2012172147 discloses an elaborate method for the production of a hop extract enriched in isoxanthohumol. Hops are dried, grinded or milled, subsequently extracted with water, dried and grinded again, and subjected to temperatures between 25 to 374 °C and pressures between 100 to 218 bars.

Although methods have been developed to produce extracts having an enhanced estrogenic activity, this activity remains relatively low and the developed methods of extracting hop phytoestrogens (8-prenylnaringenin) or their precursors are relatively complex.

### Summary of the Invention

The present invention is based on the finding that isomerisation of X (xanthohumol) into IX (isoxanthohumol) can be performed with dry spent hop material, without further extractions on the spent hop material or without addition solvents or reagents. Accordingly, a product that is considered as a waste product can still be further processed in a simple way.

A first aspect of the invention relates to methods of isomerisation of X (xanthohumol) into IX (isoxanthohumol) in a spent hop material containing polyphenols, these methods comprise the step of incubating a dry spent hop material, without further extractions on the spent hop material or without addition of solvents to the spent hop material, under inert conditions at a temperature of between 20 and 100 °C.

Herein, the inert conditions are typically a vacuum or an inert gas.

This isomerisation can be performed at a temperature of between 40 to 80°, or at a temperature between 35 to 65 °C.

The isomerisation is performed for a time period of between 2 and 30 days, or between 7 and 21 days.

Typically the isomerisation is performed until a product is obtained with a ratio of IX (w/w %) / X (xanthohumol) (w/w %) which is above 0,035, above 0,040, or above 0.050, e.g. between 0.035 to 0.060, between 0.040 to 0.060, or between 0.050 to 0.060.

In certain embodiments the spent hop material used for this method is under the form of a milled powder and/ or pellets.

The method of the present invention can be preceded by a step of milling or pelletizing the spent material under inert conditions. Typically inert conditions are maintained after the milling or pelletizing process and before the isomerisation.

In certain embodiments the isomerisation is performed in a sealed bag.

Generally spent hop material consists of the residual fraction obtained after the extraction in whole or in part of the soft resins (i.e. alpha-acids and beta-acids) and the hop oil from hops (e.g. cones, pellets, powder), and is presented under the form of a powder, pellets or the mixture thereof.

In alternative embodiments wherein said spent hop powder is mixed with a catalyst, although it is experience that efficient isomerisation can also be obtained without catalyst. Examples of catalysts are metallic divalent salts, such as magnesium oxide (MgO) or Magnesium hydroxide (MgOH), at a concentration of about 0.5% to 10.0% w/w, about 1.0% to 5.0% w/w, or about 2.0% to 4.0% of spent hop material.

Another aspect of the present invention relates to dry spent hop material comprising isoxanthohumol (IX) and xanthohumol (X) wherein the ratio of the concentration of IX (isoxanthohumol) (w/w %) over the concentration X (xanthohumol) (w/w %) is between 0,035 and 0,060, between 0.040 and 0.060, or between 0.050 and 0.060. This isomerised spent hop material can be formulated under the form of a powder, pellets or the mixture thereof.

This isomerised spent hop material can be used in the manufacture of an extract containing at least isoxanthohumol (IX).

It is an object of present invention to provide a method for the production of isomerised spent hop material further used for the production of an extract containing isoxanthohumol, further used in the treatment of physiological disorders related to disturbance in hormonal balance of estrogenic nature.

An advantage of the present invention is related to the composition of hops in the major prenylflavonoids, which is relatively poor in estrogenic compounds. The major prenylflavonoids of hop are xanthohumol (X), isoxanthohumol (IX), desmethylxanthohumol (DMX), 8-prenylnaringenin (8-PN) and 6-prenylnaringenin (6-PN). The prenylchalcones (X and DMX) are susceptible to be isomerised into prenylflavanones (IX, 8-PN and 6-PN) under certain conditions. These isomerisations are accelerated in presence of temperature and in presence of a catalyst. The estrogenic activity of hop is well known and is mainly due to the presence of 8-PN, whereas the other prenylflavonoids play a minor contribution or have no estrogenic activity. The relative composition of the different prenylflavonoids in hops varies greatly, and X is the main compound, which is present at 10 to 30 times more than 8-PN. Special attention is therefore given to xanthohumol (X) compound, which is considered in the present invention as the precursor of isoxanthohumol (IX) compound, even if the estrogenic activity of both compounds are week compared to 8-PN.

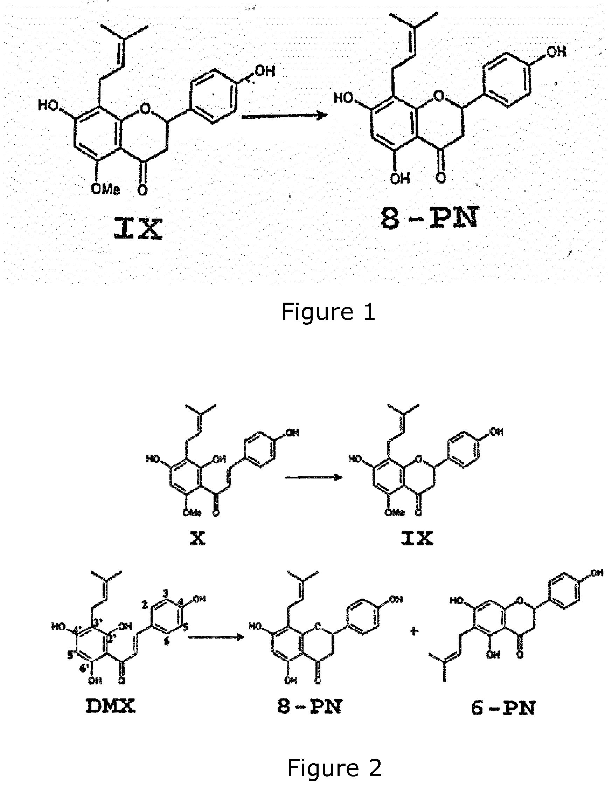

A further advantage of the present invention is the human metabolisation, as illustrated in Figure 2, of isoxanthohumol (IX), a weakly estrogenic compound into 8-prenylnaringenin (8-PN), which is the most potent estrogenic compound of hop.

Accordingly medicaments or food supplements for phytoestrogen therapy can comprise the precursor IX instead of the active compound 8-PN.

The invention describes methods of producing a new isomerised spent hop material from a spent hop material containing polyphenols is provided, the method comprising at least subjecting the spent hop material or a product derived thereof to an isomerisation, wherein the isomerised spent hop material contains at least the double content of the initial isoxanthohumol (IX) content of the spent hop material. The present invention provides new isomerised spent hop material, wherein the content in isoxanthohumol (IX) after the isomerisation, is at least the double of the initial content in IX (isoxanthohumol) present in the spent hop material used as raw materials.

The present invention describes the use of the isomerised spent hop material, for the production of an extract having a ratio isoxanthohumol (IX)/xanthohumol (X) greater than 1.

The extract is provided for the use in the treatment of physiological disorders related to disturbance in hormonal balance of estrogenic nature, such as peri-menopause or menopause.

The extract is provided for the use in the manufacture of a medicine, a cosmetic composition, a food-supplement, a food or a beverage for the treatment of physiological disorders related to disturbance in hormonal balance of estrogenic nature, such as peri-menopause or menopause.

Particular and preferred aspects of the invention are set out in the accompanying independent and dependent claims. Features from the dependent claims may be combined with features of the independent claims and with features of other dependent claims as appropriate and not merely as explicitly set out in the claims.

The present invention has the advantage that a spent hop material, under vacuum or under inert gas, can be used "as such" for the isomerisation of X (xanthohumol) into IX. Further extraction steps of the spent hop material are not required prior to the isomerisation.

Apart from the equipment to provide a spent hop material under inert conditions, no further manipulations are required or laboratory equipment is required.

The methods of the present invention merely require an incubator with temperature regulation.

The methods of the present invention have the advantage that spent hop, considered to be a waste product, is processed into a material with increased content of a phytoestrogen, with a minimum of chemicals (optional use of a catalyst), a minimum of processing steps, with minimal costs of heating, and without apparatus to generate pressures of above 100 bar.

### Detailed description of the invention

The present invention will be described with respect to particular embodiments, but the invention is not limited thereto but only by the claims.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination. In the description provided herein, numerous specific details are set forth. However, it is understood that embodiments of the invention may be practiced without these specific details. In other instances, well-known methods, structures and techniques have not been shown in detail in order not to obscure an understanding of this description.

The following terms are provided solely to aid in the understanding of the invention.

### Drawings

The figures are intended to illustrate the present invention, but should not be considered as implying any limitation of the invention to the embodiments presented therein.
- **Figure 1:**: is a schematic representation of the structure of the major prenylflavonoids and the isomerisation of the prenylchalcones (X and DMX) into prenylflavanones (IX, 8-PN and 6-PN).
- **Figure 2:**: is a schematic representation of the human O-demethylation of isoxanthohumol (IX) to form 8-prenylnaringenin (8-PN).

### Definitions

***"Spent hop material",*** as used in disclosing the present invention, means the residual solid fraction obtained from hop products (e.g. cones, pellets, powder) after an extraction with a non-polar solvent (e.g. liquid or supercritical CO₂) in whole or in part of the soft resins (i.e. α-acids and β-acids) and the hop oil. Such spent hop material contains in whole or in part, depending upon the extraction process used, the polyphenols and tannins originally present in the hop product as well as the residual fraction of vegetal material such as cellulose, lignins and ash-salts which under standard conditions is not soluble. Spent hop material is generally available in the form of a powder, pellets or mixture thereof that can be repelletized for further use, and typically contains less than 1.0 % (w/w) of alpha acids and less than 1.0 % (w/w) of beta acids, measured by HPLC according to EBC Analytica V 7.7 (ICE-3 Std).
***"Dry"*** in the context of the present context relates to a spent hop material which contains water in absolute values of less than 5 % (w/w), less than 7 % (w/w), less than 10% (w/w), of less than 12% (w/w) or less than 15 % (w/w) (weight water/weight spent hop). Typical ranges encountered in standard settings are between 7,5 - 9%, 6 to 8 %, 8 to 10%.
**"Inert"** in the context of the present invention refers to conditions wherein the oxygen (O₂) concentration, compared to ambient conditions, is dramatically reduced.
   This can be achieved by removing air by applying a vacuum or by replacing air with a gas such as nitrogen, argon or carbon dioxide. When air is replaced by an inert gas, the reaction can be performed at about atmospheric pressure.
A **"vacuum"** in the context of the present invention is sufficient when the volume of gas per volume of spent hop material is below 1/10, below 1/25, below 1/50 or below 1/100. Such vacuum can be obtained by compressing the spent hop material in a packaging such that part of the ambient air is removed, optionally further removing air by a suction pomp. When the inert conditions are obtained by replacing ambient air with a gas such as nitrogen, this is assumed to be sufficient if the concentration of oxygen in the gas, present during the isomerisation, is lowered from 20 % (v/v) in ambient air to less than 5%, less than 2% ,less than 1 %, less than 0.5 %, less than 0.1 %, less than 0.05, or even less than 0,01 %.
***"Polyphenols"*** as used in disclosing the present invention, represent approximately about 3-6% of the hop composition, and their composition depend greatly on the hop variety, the cultivation area, the harvesting technique and the degree of ageing. Polyphenols represent a vast class of compounds with widely varying structural characteristics, but having in common the presence of one or more phenolic functional groups. They comprise phenolic acids, flavanoids, flavonols, flavonol glycosides, phytoalexines and prenylflavonoids. It is generally accepted that polyphenols play an important role as anti-oxidant agent, depending on the degree of polymerisation of the polyphenols. Low-molecular weight polyphenols are natural anti-oxidants and contribute to a great extent to the protection against oxidation. It is generally recognized that the oxidation of low-molecular weight polyphenols generates a polymerization of the polyphenols concerned leading to high-molecular weight polyphenols.
***"Prenylflavonoids"*** as used in disclosing the present invention, is also called "prenylated flavonoids" and represent a sub-class of flavanoids. They are generally divided in two sub-classes, the prenylchalcones, containing xanthohumol (X) and desmethylxanthohumol (DMX), which are precursors of the prenylflavanones, containing isoxanthohumol (IX), 8-prenylnaringenin (8-PN), 6-prenylnaringenin (6-PN), 6,8-diprenylnaringenin and 8-geranylnaringenin. The major prenylflavonoids of hop are X, IX, DMX, 8-PN and 6-PN. A schematic representation of the structure of the major prenylflavonoids and the isomerisation of the prenylchalcones (X and DMX) into prenylflavanones (IX, 8-PN and 6-PN) are illustrated in Figure 1. The estrogenic activity of hop is well known and is mainly due to the presence of 8-PN, whereas the other prenylflavanones play a minor contribution and the prenylchalcones have no action. It is also known that at temperature between 40 °C and 60 °C, the isomerisation of the precursors of the prenylflavanones, namely the prenylchalcones is accelerated. Following the isomerisation, X is converted into IX and DMX is converted into a mixture of 8-PN and 6-PN. X and IX represent approximately about 80% of the prenylflavonoids present in hops.
***"Hop extraction",*** as used in disclosing the present invention, means the widely practiced extraction of hops to produce *"extract"* using solvents such as liquid and supercritical carbon dioxide and organic solvents, such as ethanol, dichloromethane, benzene, methanol, hexane and trichloroethylene. Liquid and supercritical carbon dioxide are at the moment the most efficient non-polar solvents for extracting soft resins, to produce an extract containing a high and standardized level of α-acid (between 30-60% α-acid content). The extract obtained by extraction with liquid and supercritical carbon dioxide is a product containing soft resins and hop oil with substantially no (e.g. less than 5% w/w preferably less than 1% w/w and particularly preferably less than 0.1% w/w) hard resins, substantially no (e.g. less than 10 ppm, preferably less 5 ppm) nitrate, and substantially no (e.g. less than 10 ppm, preferably less 5 ppm) polyphenols. After the extraction process, the spent hop material is considered as a waste product and is generally disposed of and there are limited utilization techniques for the brewing industry or for other alternative industries.
***"Soft resins"*** as used in disclosing the present invention, means the soluble fraction in hexane of the hop resins. They comprise two related series, the alpha-acids, also called humulones and the beta-acids, also called lupulones. To date, 5 analogues (i.e. isomers and homologues) have been characterized. The alpha-acids exhibit a very poor solubility in water and have almost no bitterness. The relative proportions of alpha-acids and beta-acids depend strongly on the hop variety and the growing conditions.
***"Hop oil",*** as used in disclosing the present invention, means a small volatile fraction (0.5 - 3.0 % w/w) of hop in which over 300 components have been positively or tentatively identified, that contribute to the hop aroma of the beer. They comprise the alcohols and esters fraction, the monoterpenes, sesquiterpenes, other terpenoids and their oxygenated derivatives fraction, and the sulphur compounds fraction.
***"Medicine"*** as used in disclosing the present invention, means a substance or a preparation used for therapeutic objective in the treatment of disease.
***"Cosmetic composition"*** as used in disclosing the present invention, means a preparation used to be applied on the human body for the purpose of beautifying, preserving, or altering the appearance of a person.
***"Food-supplement"*** as used in disclosing the present invention, means a substance or a preparation used for the maintenance of health and/or the prevention of disease.
***"Food"*** as used in disclosing the present invention, means a substance or a preparation comprising proteins, carbohydrates, lipids and supplementary substances such as minerals and vitamins used to sustain growth or to maintain the body in good conditions.
***"Beverage"*** as used in disclosing the present invention, means a liquid substance or a liquid preparation used for drinking. It comprises a lot of different liquids such as water, tea, coffee, milk, soft-drinks, beer, wine, etc.

The invention will now be described by a detailed description of several embodiments of the invention. It is clear that other embodiments of the invention can be configured according to the knowledge of persons skilled in the art, the invention being limited only by the terms of the appended claims.

### Method of producing a isomerised spend hop material

The present invention provides a method of producing a new isomerised spent hop material from a spent hop material containing polyphenols. The method comprising at least subjecting the spent hop material or a product derived thereof to an isomerisation. Herein X (xanthohumol) is converted into IX (isoxanthohumol).

The efficacy of the isomerisation can be determined by measuring the amounts (w/w %) of IX (isoxanthohumol) and X (xanthohumol) in spent hop material. During the isomerisation the concentration of X (xanthohumol) decreases whereas the concentration of IX (isoxanthohumol) increases.

Typically the isomerised spent hop material contains at least the double content of the initial isoxanthohumol (IX) content of the spent hop material.

This increase in IX (isoxanthohumol) can be assessed in the spent hop material after the isomerisation by determining the ratio between the concentration of IX (w/w %) and the concentration of X (xanthohumol) (w/w %). This value, without dimensions, is before the isomerisation between 0.01 and 0.02 and increases to between 0.030 to 0.060 (typically between 0.035 and 0.055).

Typically spent hop material containing polyphenols is collected from the extractor, after the extraction with a non-polar solvent, in whole or in part, of the soft resins and the hop oil. If the production of the isomerised hop material is delayed it is advantageous to store the spent hop material at cold temperature (preferably before 5 °C) and under vacuum or inert atmosphere to avoid oxidation of the prenylchalcones and mainly the xanthohumol (X). The collected spent hop material is relatively dry (less than 15 or 10 % (w/w) water) and generally comprises a mixture of pellets and fine particles. For further processing (e.g. addition of a catalyst), it is advantageous to mill the spent hop material to obtain a homogenous spent hop powder. During milling fine particles of spent hop material containing polyphenols become exposed to oxygen and prone to oxidation. This oxidation may affect negatively the quality of the polyphenols and may also affect negatively the quantity of prenylflavonoids and more specifically the quantity of xanthohumol available in the spent hop material. In a preferred embodiment the milling operation is performed under inert atmosphere, by flushing inert gas (e.g. CO₂, N₂) inside the mill, which will limit the oxidation and the temperature increase of the powder inside the mill. That milling is performed in any suitable mills, such as hammer mill or roller mill.

In certain embodiments, the spent hop powder is mixed with a catalyst to obtain a spent hop mixture. In a preferred embodiment the mixing operation is performed under inert atmosphere, by flushing inert gas (e.g. CO₂, N₂) inside the mill, which will limit the oxidation and the temperature increase of the powder during the mixing. That mixing is performed in any suitable devices capable of mixing powders homogeneously. This process step can be performed simultaneously during the milling step of the spent hop material. Doing so will advantageously save process time. The catalyst that is used is any suitable metallic divalent salts such as magnesium oxide (MgO) or magnesium hydroxide (MgOH) added in dry powder at a dose rate of about 0.5% to 10.0 %w/w, preferably at a dose rate of about 1.0% to 5.0 %w/w, and most preferably at a dose rate of about 2.0% to 4.0 %w/w. The concentration of the catalyst should be adapted according to the content of isoxanthohumol (IX) presents in the isomerised spent hop material obtained at the end of the isomerisation.

In another embodiment, the spent hop material, the spent hop powder or the spent hop mixture is pelletized in an appropriate pelletizing device, such as pelletizing die, to obtain spent hop pellets. Pelletizing is a critical operation and fine particles of spent hop material, spent hop powder or spent hop mixture containing polyphenols are very sensitive to oxidation. This oxidation may affect negatively the quality of the polyphenols and may also affect negatively the quantity of prenylflavonoids and more specifically the quantity of xanthohumol available in the spent hop material.

In a preferred embodiment the pelletizing operation is performed under inert atmosphere, by flushing inert gas (e.g. CO₂, N₂) inside the pelletizing device, which will limit the oxidation and the temperature increase of the powder inside the pelletizing device. The pelletizing process step is advantageous to limit the place of storage, and also the shipping costs. On the other hand the availability of the catalyst is reduced compared to a powder, which can be balanced by increasing the quantity of the catalyst during the mixing step.

Spent hop material, or any products derived thereof (i.e. spent hop powder, spent hop mixture, spent hop pellets or mixture thereof) is in the context of the present invention subjected to an isomerisation. This isomerisation is a reaction which is sensitive to oxidation. This oxidation may affect negatively the quality of the polyphenols and may also affect negatively the quantity of prenylflavonoids and more specifically the quantity of xanthohumol available in the spent hop material or any products derived thereof. It is advantageous to package the material that will be subjected to that isomerisation in any suitable packaging types such as foils or plastic bags, resistant to the temperature applied during the isomerisation. In a preferred embodiment the packaging operation is performed under vacuum or inert atmosphere, by flushing inert gas (e.g. CO₂, N₂) inside the package material, which will limit the oxidation inside the package material. Moreover, if the isomerisation is postponed it is advantageous to store the package material at cold temperature (preferably below 5 °C). If there is no packaging prior to the isomerisation this one will preferably be performed under vacuum or inert atmosphere by flushing inert gas (e.g. CO₂, N2).

The isomerisation is performed at a temperature of about 20 °C to 100 °C, preferably at a temperature of about 30 °C to 80 °C, and most preferably at a temperature of about 35 °C to 65 °C. The isomerisation occurs during a period of at least one day, preferably during a period of about 2 to 30 days, and most preferably during a period of about 7 to 21 days. In a preferred embodiment, the isomerisation is performed at constant and controlled temperature, which includes a heating phase and a cool down phase, both phases being preferably performed as fast as possible. The temperature and the duration of the isomerisation may vary in function of the concentration of the catalyst and according to the required content of isoxanthohumol (IX) presents in the isomerised spent hop material.

In a further embodiment of the first aspect according to the present invention, the resulting isomerised hop material is formulated under the form of a powder, pellets or mixture thereof. It may be advantageous to perform the isomerisation in the form of a powder, which provides a better availability of the catalyst and subsequently obtaining an improved isomerisation. The isomerised hop powder can be packaged in any suitable packaging types such as foils or plastic bags. In a preferred embodiment the packaging operation is performed under vacuum or inert atmosphere, by flushing inert gas (e.g. CO₂, N₂) inside the package material, which will limit the oxidation inside the package material. In another preferred embodiment of this invention, the isomerised hop powder will be pelletized under inert atmosphere, by flushing inert gas (e.g. CO₂, N₂) inside the pelletizing device, which will limit the oxidation and the temperature increase of the powder inside the pelletizing device. In a preferred embodiment the packaged isomerised hop material is stored at cold temperature (preferably below 5 °C), to avoid oxidation before further use of the isomerised hop material.

### Isomerised spent hop material

In a second aspect of the present invention, a new isomerised spent hop material is provided, wherein the content in isoxanthohumol (IX) after the isomerisation as described in the first aspect of this invention, is at least the double of the initial content in IX present in the spent hop material used as raw materials. This can be measured by determining the ration of IX versus X (xanthohumol) after the isomerisation. The isomerised spent hop material may be under the form of a powder, pellets or mixture therefore according to the form of the raw material used during the isomerisation.

### Uses of isomerised spent hop material

In a third aspect of the present invention, the use of a new isomerised spent hop material is provided, for the production of an extract having a ratio isoxanthohumol (IX)/ xanthohumol (X) greater than 1. This extract is obtainable by any suitable process capable of extracting the prenylflavonoids compounds and mainly the isoxanthohumol (IX) present in the isomerised spent hop material produced according the isomerisation method described in the first aspect of this invention. The extract containing isoxanthohumol (IX) can be a liquid or a powder in function of the extraction process that is used to obtain the extract.

In a first embodiment of the third aspect according to the present invention, the extract is used in the treatment of physiological disorders related to disturbance in hormonal balance of estrogenic nature, such as peri-menopause or menopause. Major symptoms, complaints or diseases caused by disturbance in hormonal balance of estrogenic nature are mainly, but not limited to hot flushes, osteoporosis, heart diseases, sex-hormone-dependant cancer such as breast cancer.

In a second embodiment of the third aspect of the present invention, the extract is used in the manufacture of a medicine, a cosmetic composition, a food-supplement, a food or a beverage for the treatment of physiological disorders related to disturbance in hormonal balance of estrogenic nature, such as peri-menopause or menopause.

The present invention is further elucidated in the following examples and tables. Three types of spent hop materials from American hops were investigated for their content in prenylflavonoids.

### Example 1: Analyses of prenylflavonoids in spent hops material from extractor

The first type of spent hop material (SPE-TBCTZ019T) was the control and corresponds to the spent hop material collected after a supercritical liquid CO₂ extraction process.

### Method of Analysis

An amount of 3.5-4.5 g of spent hop material was carefully weighed into an extraction vessel and ca. 40-50 mL of methanol was added (weight of methanol was recorded). Extraction was assisted by sonication at 35 °C for 30 min. Samples were homogenized before filtration over 0.2 µm (RC-filters) into a vial for HPLC analysis.

HPLC-analysis was carried out using a Waters 2695 Alliance Separation Module equipped with a 996 Photodiode Array. Gradient elution was carried out on a C-18 column (Waters XTerra) and the injection volume was 20 µL. Calibration was done using the external standards methods (all standards were prepared by preparative HPLC and quality control was done by 1H NMR). Prenylflavanones [isoxanthohumol (IX), 8-prenylnaringenin (8-PN), and 6-prenylnaringenin (6-PN)] were detected at 295 nm. Prenylchalcones [desmethylxanthohumol (DMX) and xanthohumol (X)] were detected at 370 nm. Peak integration was automatically performed by the Waters Millenium software and peak areas were used for quantification purposes.

The results are expressed in w/w% and presented in Table 1.

**Table 1:**

| **Spent Hop Samples** | **X (% w/w)** | **IX (% w/w)** | **IX/X** | **DMX (% w/w)** | **8-PN (% w/w)** | **6-PN (% w/w)** |
|---|---|---|---|---|---|---|
| **Sample 1** | 1.248 | 0.017 | 0.014 | 0.097 | 0.013 | 0.025 |
| **Sample 2** | 1.279 | 0.018 | 0.014 | 0.103 | 0.014 | 0.026 |
| **Sample 3** | 1.254 | 0.017 | 0.014 | 0.098 | 0.014 | 0.024 |
| | | | | | | |
| **Mean** | **1.260** | **0.017** | | **0.099** | **0.014** | **0.025** |
| **St. Dev.** | **0.016** | **0.001** | | **0.003** | **0.001** | **0.001** |

As shown in Table 1, The spent hops material showed a typical chromatographic profile with X and DMX as the major hop-derived prenylflavonoids. In addition to the prenylchalcones (X and DMX), also significant amounts of prenyflavanones (IX, 8-PN and 6-PN) could be detected. However, the concentration of the prenylflavanones is significantly lower than the concentration of the prenylchalcones.

### Example 2: Analyses of prenylflavonoids in spent hops material exposed to high temperature

The second type of spent hop material (SPE-TBCTZ019) was the same material as presented in example 1, which was subjected at a temperature of about 50 °C during 2 weeks.

The analysis of_prenylflavonoids was performed by using the method presented in example 1. The results are expressed in w/w% and presented in Table 2.

**Table 2:**

| **Spent Hop Samples** | **X (% w/w)** | **IX (% w/w)** | ***IX*/*X*** | **DMX (% w/w)** | **8-PN (% w/w)** | **6-PN (% w/w)** |
|---|---|---|---|---|---|---|
| **Sample 1** | **1.137** | **0.044** | *0.039* | 0.020 | 0.018 | 0.054 |
| **Sample 2** | **1.178** | **0.046** | *0.039* | 0.018 | 0.018 | 0.052 |
| **Sample** 3 | **1.180** | **0.043** | *0.036* | 0.019 | 0.018 | 0.047 |
| **Mean** | **1.165** | **0.044** | | **0.019** | **0.018** | **0.051** |
| **St. Dev.** | **0.024** | **0.001** | | **0.001** | **0.001** | **0.003** |

As shown in Table 2, the spent hops material showed a typical chromatographic profile, which is indicative of exposure to high temperature. The content in prenylchalcones (X and DMX) is significantly reduced compared to the results presented in Table 1. In addition the amounts of prenylflavanones (IX, 8-PN and 6-PN) are significantly increased compared to the results presented in Table 1. These modifications of the composition of the spent hop material are due to the isomerisations that occur at high temperature conditions.

### Example 3: Analyses of prenylflavonoids in spent hops material exposed to high temperature in presence of a catalyst

The third type of spent hop material (SPE-TBCTZ020) was the same material as presented in example 1, which was subjected at a temperature of about 50 °C during 2 weeks, in presence of 3% (w/w) of MgO as catalyst.

The analyses of prenylflavonoids were performed by using the method presented in example 1. The results are expressed in % w/w and presented in Table 3.

**Table 3:**

| **Spent Hop Samples** | **X (% w/w)** | **IX (% w/w)** | ***IX*/*X*** | **DMX (% w/w)** | **8-PN (% w/w)** | **6-PN (% w/w)** |
|---|---|---|---|---|---|---|
| **1** | **1.133** | **0.049** | *0.043* | 0.005 | 0.018 | 0.055 |
| **2** | **1.113** | **0.049** | *0.043* | 0.005 | 0.019 | 0.055 |
| **3** | **1.115** | **0.050** | *0.045* | 0.006 | 0.019 | 0.056 |
| | | | | | | |
| **Mean** | **1.120** | **0.049** | | **0.006** | **0.019** | **0.055** |
| **St. Dev.** | **0.011** | **0.001** | | **0.001** | **0.001** | **0.001** |

As shown in Table 3, the spent hops material showed a typical chromatographic profile, which is indicative of exposure to high temperature. The content in prenylchalcones (X and DMX) is slightly reduced compared to the results presented in Table 2. In addition the amounts of prenylflavanones (IX, 8-PN and 6-PN) are slightly increased compared to the results presented in Table 2. These modifications of the composition of the spent hop material show a limited effect of the catalyst on the isomerisation. However the residual content of DMX is very, which means that the isomerisation of DMX into 8-PN and 6-PN is nearly completed. On the contrary, the content of residual X is still high, which means that the isomerisation of X into IX is not completed, and that the content in IX can be much higher under optimized reaction conditions.

### Example 4: Analyses of prenylflavonoids in spent hops material and Isomerised spent hop material from different varieties

The analyses of prenylflavonoids were performed by using the method presented in example 1. Different varieties of hop (i.e. CTZ, Bravo, Millenium, Galena, Nugget and Summit) were evaluated for their respective content in prenylflavonoids. These analyses were performed on spent hop material (N) and isomerised spent hop material (T), obtained without catalyst after an exposure during 21 days at 47 °C, from the same batch for each variety. The results are expressed in % w/w and presented in Table 4.

**Table 4:**

| Variety* | | **IX** | 8-PN | DMX | 6-PN | **X** | *IX*/*X* | IX (T/N) | 8-PN (T/N) |
|---|---|---|---|---|---|---|---|---|---|
| CTZ | N | **0,014** | 0,006 | 0,077 | 0,017 | **1,149** | *0.012* | | |
| | T | **0,058** | 0,013 | 0,010 | 0,036 | **1,096** | ***0.052*** | 4,143 | 2,167 |
| BRAVO | N | **0,014** | 0,003 | 0,061 | 0,015 | **1,300** | *0.011* | | |
| | T | **0,049** | 0,009 | 0,016 | 0,030 | **1,284** | ***0.038*** | 3,500 | 3,000 |
| MILLENIUM | N | **0,014** | 0,009 | 0,130 | 0,032 | **0,960** | *0.015* | | |
| | T | **0,040** | 0,019 | 0,042 | 0,049 | **0,898** | ***0.044*** | 2,857 | 2,111 |
| GALENA | N | **0,012** | 0,009 | 0,112 | 0,037 | **0,801** | *0.014* | | |
| | T | **0,035** | 0,019 | 0,029 | 0,058 | **0,723** | ***0.048*** | 2,917 | 2,111 |
| NUGGET | N | **0,010** | 0,007 | 0,085 | 0,023 | **0,707** | *0.014* | | |
| | T | **0,029** | 0,014 | 0,028 | 0,037 | **0,699** | ***0.056*** | 2,900 | 2,000 |
| SUMMIT | N | **0,006** | 0,011 | 0,085 | 0,035 | **0,424** | *0.014* | | |
| | T | **0,014** | 0,019 | 0,024 | 0,058 | **0,399** | ***0.035*** | 2,333 | 1,727 |
| Average | | | | | | | | 3,108 | 2,186 |

| | | | | | | | | | |
|---|---|---|---|---|---|---|---|---|---|
| N = Normal (no treatment) T= Treated (thermal treatment: 21 days @ 47 °C) | | | | | | | | | |

As shown in Table 4, there are significant changes between the prenylflavonoids content of the spent hop material (N) and of the isomerised spent hop material. The content in prenylchalcones (X and DMX) is significantly reduced and the amounts of prenylflavanones (IX, 8-PN and 6-PN) are significantly increased in T samples compared to N samples. These results confirm the results obtained in example 1 and example 2. The average increase in IX is about 3 times more in the treated samples (T) and the increase of 8-PN is about twice. It is also shown that there are some varietal differences in the prenylflavonoids content, where varieties CTZ, Bravo and Millenium contain more than Galena, Nugget and Summit.

### Example 5: Extract produced from spent hops material

A quantity of about 5 kg of spent hop material (SPE-TBCTZ019) from example 2 was used in a specific extraction process for the production of about 45 g of a powder extract.

The extract was analysed by HPLC and the results are expressed in w/w%, as presented in Table 5.

**Table 5:**

| **X (% w/w)** | **IX (% w/w)** | **IX/X** | **8-PN (% w/w)** | **6-PN (% w/w)** | **IX/X Ratio** |
|---|---|---|---|---|---|
| **21.6** | **33** | **1.53** | **1.1** | **3.2** | **1.53** |

As shown in Table 5, the concentration of the extract in IX is particularly high, which allow a ratio IX/X greater than 1.0 and in this case around 1.5.

## Claims

1. A method of isomerisation of X (xanthohumol) into IX (isoxanthohumol), the method comprising the step of incubating dry spent hop material containing polyphenols, without further extractions on the spent hop material or without addition of solvents to the spent hop material, under inert conditions at a temperature of between 20 and 100 °C, until a product is obtained with a ratio of IX (w/w%) / X (xanthohumol) (w/w%) which is above 0,035.

2. The method according to claim 1, wherein the ratio IX/X is above 0,040.

3. The method according to claim 1, wherein the ratio IX/X is above 0,050.

4. The method according to any one of claims 1 to 3, wherein said dry spent hop material is the residual fraction obtained after the extraction in whole or in part of soft resins and hop oil from hops and wherein said dry spent hop material is kept under inert conditions in the period after the extraction and before the isomerisation.

5. The method according to any one of claims 1 to 4, wherein the inert conditions are a vacuum or an inert gas.

6. The method according to any one of claims 1 or 5, wherein the isomerisation is performed at a temperature of between 40 to 80 °C.

7. The method according to any one of claims 1 to 5, wherein the isomerisation is performed at a temperature between 35 to 65 °C.

8. The method according to any one of claims 1 to 7, wherein the isomerisation is performed for a time period of between 2 and 30 days.

9. The method according to any one of claims 1 to 7, wherein the isomerisation is performed for a time period of between 7 and 21 days.

10. The method according to any one of claims 1 to 9, wherein the dry spent hop material used for this method is a milled product.

11. The method according to claim 10, wherein said milling is performed under inert conditions.

12. The method according to any one of claims 1 to 11, wherein the isomerisation is performed in a sealed bag.

13. The method according to any one of claims 1 to 12, wherein said dry spent hop is mixed with a catalyst, such as magnesium oxide (MgO) or Magnesium hydroxide (MgOH).

14. Dry spent hop material comprising isoxanthohumol (IX) and xanthohumol (X) **characterised in that** the ratio of the concentration of IX (isoxanthohumol) (w/w %) over the concentration X (xanthohumol) (w/w %) is between 0,035 and 0,060.

15. Use of dry spent hop material according to claim 14 for the manufacture of an extract containing at least isoxanthohumol (IX).

## Patentansprüche

1. Verfahren zum Isomerisieren von X (Xanthohumol) in IX (Isoxanthohumol), wobei das Verfahren den Schritt zum Inkubieren von trockenem verbrauchten Hopfenmaterial umfasst, das Polyphenole enthält, ohne weitere Extraktionen des verbrauchten Hopfenmaterials oder ohne Zugabe von Lösemitteln zum dem verbrauchten Hopfenmaterial, unter inerten Bedingungen bei einer Temperatur zwischen 20 und 100°C, bis ein Produkt mit einem Verhältnis von IX (w/w%)/X (Xanthohumol) (w/w%) erhalten wird, das über 0,035 ist.

2. Verfahren nach Anspruch 1, wobei das Verhältnis IX/X über 0,040 ist.

3. Verfahren nach Anspruch 1, wobei das Verhältnis IX/X über 0,050 ist.

4. Verfahren nach einem der Ansprüche 1 bis 3, wobei das trockene verbrauchte Hopfenmaterial die Restfraktion ist, die nach Extraktion im Ganzen oder teilweise von weichen Harzen und Hopfenölen aus Hopfen erhalten wird, und wobei das trockene verbrauchte Hopfenmaterial in dem Zeitraum nach der Extraktion und vor der Isomerisierung unter inerten Bedingungen gehalten wird.

5. Verfahren nach einem der Ansprüche 1 bis 4, wobei die inerten Bedingungen ein Vakuum oder ein inertes Gas sind.

6. Verfahren nach einem der Ansprüche 1 bis 5, wobei die Isomerisierung bei einer Temperatur zwischen 40 und 80°C ausgeführt wird.

7. Verfahren nach einem der Ansprüche 1 bis 5, wobei die Isomerisierung bei einer Temperatur zwischen 35 und 65°C ausgeführt wird.

8. Verfahren nach einem der Ansprüche 1 bis 7, wobei die Isomerisierung für einen Zeitraum zwischen 2 und 30 Tagen ausgeführt wird.

9. Verfahren nach einem der Ansprüche 1 bis 7, wobei die Isomerisierung für einen Zeitraum zwischen 7 und 21 Tagen ausgeführt wird.

10. Verfahren nach einem der Ansprüche 1 bis 9, wobei das trockene verbrauchte Hopfenmaterial, das für dieses Verfahren verwendet wird, ein gemahlenes Produkt ist.

11. Verfahren nach Anspruch 10, wobei das Mahlen unter inerten Bedingungen durchgeführt wird.

12. Verfahren nach einem der Ansprüche 1 bis 11, wobei die Isomerisierung in einem versiegelten Beutel durchgeführt wird.

13. Verfahren nach einem der Ansprüche 1 bis 12, wobei das trockene verbrauchte Hopfenmaterial mit einem Katalysator, wie Magnesiumoxid (MgO) oder Magnesiumhydroxid (MgOH) gemischt wird.

14. Trockenes verbrauchtes Hopfenmaterial, umfassend Isoxanthohumol (IX) und Xanthohumol (X), **dadurch gekennzeichnet, dass** das Verhältnis der Konzentration von IX (Isoxanthohumol) (w/w%) gegenüber der Konzentration von X (Xanthohumol) (w/w%) zwischen 0,035 und 0,060 liegt.

15. Verwendung von trockenem verbrauchten Hopfenmaterial nach Anspruch 14 für die Herstellung eines Extrakts, das mindestens Isoxanthohumol (IX) enthält.

## Revendications

1. Procédé d'isomérisation de X (xanthohumol) en IX (isoxanthohumol), le procédé comprenant l'étape d'incubation de matière sèche de drêche de houblon contenant des polyphénols, sans extractions supplémentaires sur la matière de drêche de houblon ou sans ajout de solvants à la matière de drêche de houblon, sous des conditions inertes à une température entre 20 et 100 °C, jusqu'à ce qu'un produit soit obtenu avec un rapport d'IX (% en M/M) / X (xanthohumol) (% en M/M) qui est supérieur à 0,035.

2. Procédé selon la revendication 1, dans lequel le rapport IX/X est supérieur à 0,040.

3. Procédé selon la revendication 1, dans lequel le rapport IX/X est supérieur à 0,050.

4. Procédé selon l'une quelconque des revendications 1 à 3, dans lequel ladite matière sèche de drêche de houblon est la fraction résiduelle obtenue après l'extraction en totalité ou en partie de résines molles et d'huile de houblon à partir de houblons et dans lequel ladite matière sèche de drêche de houblon est conservée sous des conditions inertes dans la période après l'extraction et avant l'isomérisation.

5. Procédé selon l'une quelconque des revendications 1 à 4, dans lequel les conditions inertes sont un vide ou un gaz inerte.

6. Procédé selon l'une quelconque des revendications 1 ou 5, dans lequel l'isomérisation est effectuée à une température entre 40 et 80 °C.

7. Procédé selon l'une quelconque des revendications 1 à 5, dans lequel l'isomérisation est effectuée à une température entre 35 et 65 °C.

8. Procédé selon l'une quelconque des revendications 1 à 7, dans lequel l'isomérisation est effectuée pendant une période de temps entre 2 et 30 jours.

9. Procédé selon l'une quelconque des revendications 1 à 7, dans lequel l'isomérisation est effectuée pendant une période de temps entre 7 et 21 jours.

10. Procédé selon l'une quelconque des revendications 1 à 9, dans lequel la matière sèche de drêche de houblon utilisée pour ce procédé est un produit broyé.

11. Procédé selon la revendication 10, dans lequel ledit broyage est effectué sous des conditions inertes.

12. Procédé selon l'une quelconque des revendications 1 à 11, dans lequel l'isomérisation est effectuée dans un sac scellé.

13. Procédé selon l'une quelconque des revendications 1 à 12, dans lequel ladite drêche de houblon sèche est mélangée avec un catalyseur, tel que de l'oxyde de magnésium (MgO) ou de l'hydroxyde de Magnésium (MgOH).

14. Matière sèche de drêche de houblon comprenant de l'isoxanthohumol (IX) et du xanthohumol (X) **caractérisé en ce que** le rapport de la concentration d'IX (isoxanthohumol) (% en M/M) sur la concentration de X (xanthohumol) (% en M/M) est entre 0,035 et 0,060.

15. Utilisation de matière sèche de drêche de houblon selon la revendication 14 pour la fabrication d'un extrait contenant au moins de l'isoxanthohumol (IX).
